# FASCICULE DE BREVET EUROPEEN

(11) **EP 2 414 001 B1**
(45) Date de publication et mention de la délivrance du brevet: **13.01.2016**
(21) Numéro de dépôt: 09784050.8
(22) Date de dépôt: 16.10.2009
(51) Int. Cl.: A61M 5/20, A61M 5/24, A61M 5/32, A61M 5/315, G06F 19/00

(54) **DISPOSITIF D'INJECTION AUTOMATIQUE**
AUTOMATISCHE EINSPRITZVORRICHTUNG
AUTOMATIC INJECTION DEVICE

(30) Priorité: 02.04.2009 EP 09157227
(43) Date de publication de la demande: 08.02.2012
(73) Titulaire: Pongpairochana, Vincent, 1093 La Conversion (CH)
(72) Inventeur: Pongpairochana, Vincent, 1093 La Conversion (CH)
(74) Mandataire: GLN SA
(86) Numéro de dépôt international: PCT/EP2009/063598
(87) Numéro de publication internationale: WO 2010/112090

(56) Documents cités:
- EP-A1- 0 108 529
- WO-A1-2004/098687
- WO-A2-2005/077441

## Description

### Domaine technique

La présente invention concerne un dispositif d'injection, doté d'un compartiment articulé en référence au dispositif pour recevoir une seringue munie d'une aiguille protégée par un capuchon de protection solidaire de la seringue. Le compartiment est susceptible d'évoluer entre une première position ouverte pour l'insertion et le retrait de la seringue, et une deuxième position fermée pour la réalisation de l'injection.

### Etat de la technique

De nombreux traitements nécessitent que la patient subisse des injections intramusculaires quotidiennes ou sous-cutanées fréquentes et à intervalles régulières, mensuels, hebdomadaires. Il est courant, pour permettre au patient de mener une vie normale, que celui-ci se fasse lui-même les injections. Dans ce but, plusieurs dispositifs ont été mis au point, avec notamment pour objectif de faciliter l'opération d'injection, tant du point de vue ergonomique que psychologique. Au sujet de ce dernier point, on peut aisément concevoir que la vision de l'aiguille peut faire peur au patient. De plus, le risque de blessure ou de contamination est crucial, notamment pour les personnes tierces devant manipuler les dispositifs.

Pour améliorer la sécurité des seringues de type classique, il a été développé des capuchons, passifs ou actifs, permettant de cacher l'aiguille. Néanmoins, ce genre de capuchons ne procure aucune amélioration de la fonctionnalité du dispositif d'auto-injection et c'est toujours au patient, comme pour une seringue conventionnelle, d'effectuer l'action motrice pour réaliser l'injection.

Des dispositifs d'auto-injection proposent de suppléer le patient pour effectuer automatiquement l'injection, après le déclenchement de celle-ci. Il existe ainsi des dispositifs jetables. Leur coût est un inconvénient majeur dans le cas d'un traitement impliquant de fréquentes injections. En outre, étant donné que le dispositif est à usage unique, il est très peu perfectionné techniquement, notamment d'un point de vue contrôle, ou au niveau de moyens électroniques permettant de perfectionner les fonctionnalités.

Des dispositifs utilisent parfois des seringues conventionnelles, comme proposé dans le document WO2004/098687, ce qui est avantageux, car cela permet de combiner des éléments jetables pour les parties en contact avec le médicament, et des éléments pouvant être perfectionnés, car étant conservés d'une utilisation à une autre. Les protocoles de traitement sont généralement conçus pour que le contenu de la seringue soit injecté entièrement en une seule fois. Cependant, il est nécessaire d'effectuer beaucoup de manipulation, avant l'injection, pour retirer le capuchon protégeant la seringue, introduire la seringue dans le dispositif, puis, après l'injection, retirer la seringue du dispositif et remettre le capuchon. Ce genre de dispositifs présente l'inconvénient de devoir effectuer certaines de ces manipulations alors que l'aiguille n'est pas protégée. Les manipulations de la seringue usagée et contaminée après injection exposent le personnel hospitalier ou l'entourage du patient à des risques d'infection.

Un autre type de dispositif propose d'utiliser des cartouches multidoses de médicament, c'est-à-dire susceptibles d'être utilisées plusieurs fois consécutivement, l'appareil se chargeant d'administrer la bonne quantité au patient. Ce genre d'appareil peut être bien sécurisé pour le patient et les personnes devant l'avoir entre les mains, car l'aiguille d'injection est toujours cachée et ne présente pas de risque de blessure. Un tel appareil est particulièrement sophistiqué, notamment pour assurer de manière sûre le dosage du médicament, mais également pour assurer sa programmation, en fonction du traitement et du médicament. Ces appareils sont adaptés pour des cartouches multidoses, mais ne peuvent recevoir des seringues conventionnelles.

La présente invention a pour but de proposer un dispositif permettant de combiner les avantages des appareils ci-dessus, en évitant les inconvénients. Particulièrement, l'invention porte sur un dispositif très sûr, avantageux au niveau de l'impact sur le coût du traitement et pouvant être couplé à une électronique performante.

### Divulgation de l'invention

De manière plus précise, l'invention porte sur un dispositif d'auto-injection doté d'un compartiment articulé en référence au dispositif pour recevoir une seringue munie d'une aiguille protégée par un capuchon de protection solidaire de la seringue. Le compartiment est susceptible d'évoluer entre une première position ouverte pour l'insertion et le retrait de la seringue, et une deuxième position fermée pour la réalisation de l'injection. Selon l'invention et afin d'éviter tout risque de blessure ou de contamination avec l'aiguille, le dispositif comporte un système de désolidarisation destiné à coopérer avec le capuchon de la seringue pour le désolidariser de la seringue, la désolidarisation étant conditionnée par le passage du compartiment à sa deuxième position. En outre, le passage du compartiment de la deuxième à la première position est conditionné par la solidarisation du capuchon.

L'invention concerne également un système de gestion et de contrôle de protocoles d'injection, comprenant :
- un serveur stockant des données relatives au patient et à son traitement, et
- au moins un dispositif selon l'une des revendications précédentes pour réaliser les injections, comportant des moyens de connexion pour être relié à distance au serveur et des moyens électroniques,
   caractérisé en ce que lesdits moyens électroniques sont agencés pour
- enregistrer l'historique des injections,
- suivre et contrôler le suivi du traitement et, éventuellement, rappeler au patient le moment des injections,
- transmettre au serveur ledit historique,
- recevoir et traiter des informations transmises par le serveur.

D'autres aspects de l'invention sont mentionnés dans les revendications de la présente demande.

### Brève description des dessins

D'autres caractéristiques de la présente invention apparaîtront plus clairement à la lecture de la description qui va suivre, faite en référence au dessin annexé, dans lequel:
- les figures 1 à 9 représentent différentes étapes successives de la mise en oeuvre d'un dispositif selon l'invention, les figures 1b et 2b étant des vues agrandies, et
- la figure 10 schématisant un système de gestion et de contrôle de protocoles d'injection,
- la figure 11 illustre un exemple de marquage de reconnaissance d'une seringue, particulièrement adapté à un dispositif selon l'invention, et
- les figures 12 à 15 montrent différentes étapes successives de la mise en oeuvre d'un dispositif selon un autre mode de réalisation de l'invention.

### Mode(s) de réalisation de l'invention

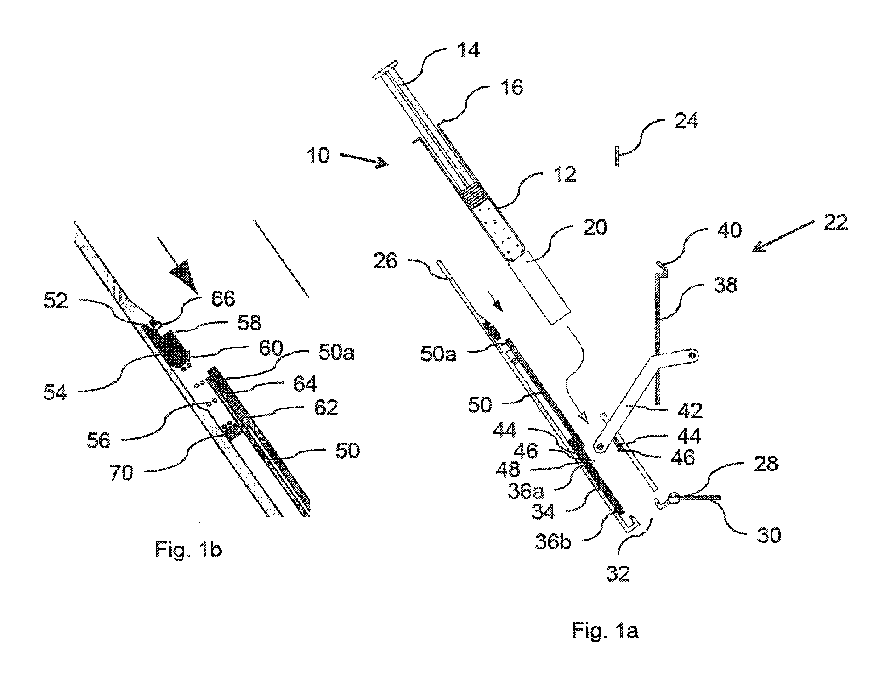

Les figures 1 à 9 représentent, de manière schématique, un dispositif permettant à un patient de s'auto-injecter le contenu d'une seringue 10 de type conventionnel. On entend par seringue conventionnelle, une seringue comprenant un corps 12 à l'intérieur duquel peut prendre place un liquide, pouvant être comprimé par un piston 14 mobile à l'intérieur du corps. En général, l'extrémité du corps 12 recevant le piston est muni d'une collerette 16, pour fournir un appui pour une injection manuelle. La seringue 10 peut recevoir, à son extrémité opposée à la collerette, une aiguille 18 creuse, pour injecter le liquide contenu dans le corps 12 de la seringue 10, dans le patient.

Pour protéger l'aiguille 18, celle-ci est recouverte, lorsque la seringue 10 n'est pas utilisée, d'un capuchon 20, en général réalisé en plastique rigide, solidarisé en force avec la seringue 10.

Sur les figures 1 à 9, le dispositif est représenté de manière schématique, afin de rendre plus claire la compréhension de l'invention. Ainsi, les éléments électroniques régissant l'injection, les moteurs et autres écrans pour l'affichage d'information, ne sont pas représentés sur les dessins. Pour ne pas surcharger les dessins, certains éléments n'ont été représentés que sur certaines figures.

Ainsi, la figure 1 montre un dispositif d'auto-injection 22 selon l'invention, dont une première paroi 24 comporte un volet 26 articulé sur une charnière 28. Cette dernière est, de préférence, située sur une deuxième paroi 30, adjacente à la première 24. On définit de la sorte un compartiment, articulé en référence au dispositif 22. Comme on va le comprendre par la suite, ce compartiment est destiné à recevoir une seringue 10, pour l'injection de son contenu.

La deuxième paroi 30 est percée, dans sa partie appartenant au compartiment, d'une ouverture 32 pour le passage de l'aiguille 18 de la seringue 10 et de son capuchon 20. Pour recevoir cette dernière, le compartiment comporte encore un anneau de guidage 34, solidaire du volet 26 et situé du côté de l'ouverture 32, de manière à guider la seringue 10 afin qu'elle soit dans la position souhaitée pour son insertion dans le compartiment et pour l'injection. L'anneau de guidage 34 présente, sur sa paroi intérieure, une première 36a et une deuxième 36b creusures, alignées selon l'axe longitudinal du compartiment et éloignées l'une de l'autre. Les creusures 36a et 36b sont identiques et forment une série. Plusieurs séries peuvent être réparties sur la circonférence de la paroi intérieure de l'anneau de guidage. Le rôle de ces creusures 36a et 36b apparaîtra plus loin dans la description.

Le dispositif comporte encore une cloison interne 38 située dans le dispositif, afin de parfaitement délimiter un logement pour la seringue 10. Cette cloison interne 38 présente un épaulement 40 pour coopérer avec la collerette 16 de la seringue 10. La cloison interne 38 est agencée de manière à prolonger l'anneau de guidage 34 lorsque le compartiment est dans une position fermée, c'est-à-dire lorsque le volet 26 est aligné sur la première paroi 24.

Un bras 42 est également monté pivotant à une première extrémité sur le dispositif. Une deuxième extrémité du bras 42 est montée pivotante sur le compartiment, pour tenir le volet 26 et définir une position d'ouverture maximale. Sont ainsi définies une première position d'ouverture maximale pour l'insertion et le retrait de la seringue, et une deuxième position fermée pour la réalisation de l'injection.

Selon un point intéressant de l'invention, le compartiment comprend des moyens de désolidarisation du capuchon en référence à la seringue. Ces moyens peuvent être formés par une bague 44 ajustée à l'intérieur de l'anneau de guidage 34. La deuxième extrémité du bras 42 est pivotée sur la bague 44, de sorte que l'ouverture et la fermeture du volet 26 en référence au dispositif, entraînent un mouvement de translation de la bague 44, respectivement en direction de l'ouverture 32 et en s'éloignant de l'ouverture 32.

Plus précisément, le diamètre extérieur de la bague 44 est légèrement inférieur au diamètre intérieur de l'anneau de guidage 34, de manière à ce que leur paroi, respectivement extérieure et intérieure, soient en contact l'une avec l'autre. La bague 44 comporte un ou plusieurs crochets 46, situés de préférence à l'extrémité de la bague 44. Les crochets 46 sont dotés d'un renflement 48 situé sur la paroi extérieure de la bague 44 et de forme complémentaire aux creusures 36a et 36b. Chaque crochet est destiné à coopérer avec les creusures d'une série. La figure 1a montre deux crochets 46, positionnés à des hauteurs différentes en référence au compartiment et, comme on le comprendra ci-après, destinés à coopérer différemment avec un capuchon 20.

La course de l'anneau de guidage 34 définie par la géométrie du bras 42, est égale à la distance séparant deux creusures 36a et 36b d'une série. Les crochets 46 sont positionnés de manière à ce que, lorsque le compartiment est dans sa première position d'ouverture maximale, les renflements 48 sont logés dans les premières creusures 36a et lorsque le compartiment est dans sa deuxième position fermée, les renflements 48 sont logés dans les deuxièmes creusures 36b. Naturellement, entre ces deux positions, les crochets 46 translatent entre les deux creusures 36a et 36b et les renflements 48 sont appuyés sur la paroi intérieure de l'anneau de guidage 34, ce qui contraint légèrement la bague de guidage 34 et rapproche les crochets 46 du centre de la bague de guidage 34. La forme des renflements 48 et les creusures 36 est adaptée pour ne pas avoir de blocage entre ces éléments.

On notera également que la bague de guidage 34 peut comporter un prolongement 50 situé du côté du volet, doté d'un doigt 50a dont le rôle apparaîtra plus loin.

Le dispositif d'auto-injection 22 est encore doté d'un système de verrouillage du volet 26, non représenté. Ce système permet que le passage du compartiment de sa deuxième position de fermeture à une position d'ouverture, soit conditionné par la solidarisation du capuchon 20 en référence à la seringue 10. Ainsi, pour sécuriser l'utilisation du dispositif d'auto-injection et garantir qu'une seringue 10 ne peut être extraite du compartiment, particulièrement après une injection, que si le capuchon 20 de sécurité a été remis en place, un capteur, par exemple de type optique, peut être disposé dans le compartiment afin de détecter la présence du capuchon 20, à une hauteur signifiant qu'il est nécessairement solidarisé avec la seringue 10. Ce capteur peut être avantageusement relié à un loquet, non représenté, coopérant avec le volet 26 lorsque celui-ci est fermé. Le loquet peut être commandé électriquement de manière à être verrouillé et à ne pouvoir libérer le volet 26 que si le signal transmis pas le capteur indique que le capuchon 20 est bien en place sur la seringue 10. Naturellement, d'autres types de capteurs peuvent être utilisés pour détecter la présence du capuchon. Un système de verrouillage complètement mécanique pourrait également être envisagé.

De manière avantageuse, le dispositif d'auto-injection représenté au dessin comporte également un système de verrouillage de la seringue 10 dans le compartiment, qui permet, lorsque la seringue 10 a été complètement insérée dans le compartiment, de ne faire ressortir la seringue 10 que lorsque le volet 26 est complètement ouvert.

Dans ce but, la paroi intérieure du volet 26 comprend un ergot 52, formant butée pour un chariot 54, susceptible de coulisser le long de la paroi intérieure du volet 26. Un premier ressort 56 exerce une force sur le chariot 54 tendant à l'amener contre l'ergot 52. Le chariot 54 comporte encore un décrochement 58 agencé de manière à ce que la seringue, plus particulièrement sa collerette 16, puisse prendre appui sur lui, lorsqu'elle est insérée dans le compartiment. Le chariot 54 comporte encore un bec 60 monté rétractable, s'étendant en direction de l'intérieur du compartiment. L'intérieur du compartiment comporte encore une deuxième cloison interne 62, ajustée à l'intérieur de la bague 44 et comprenant sur sa paroi extérieure, une échancrure 64 de forme complémentaire au bec 60. Ce dernier est agencé de manière ce que le doigt 50a du prolongement 50 le fasse se rétracter lorsqu'ils se croisent dans une direction, en l'occurrence, lorsque le prolongement 50 se déplace en direction de l'extrémité du volet. Lorsque le prolongement 50 se déplace dans une autre direction, le bec est agencé de manière à ce que le doigt 50a puisse échapper du bec 60, sans qu'il y ait d'interaction entre ces éléments. Le bec 60 est positionné de manière à pouvoir coopérer avec l'échancrure 64 pour former des moyens de blocage.

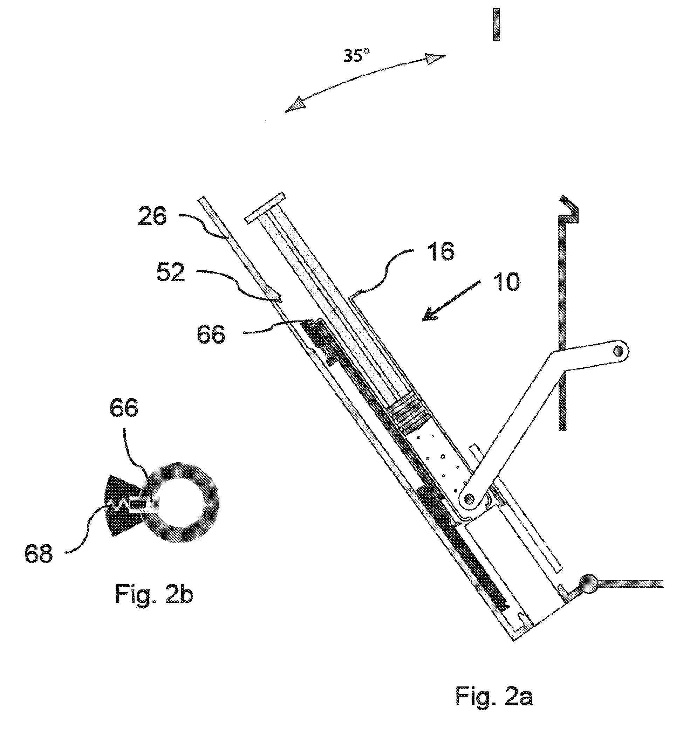

Le chariot 54 présente encore, au niveau du décrochement 58, une languette 66, mieux visibles sur la figure 2b. La languette 66 est mobile radialement et coopère avec un deuxième ressort 68, moins fort que le premier, exerçant une force tendant à l'entraîner vers le milieu du compartiment. L'ergot 52 est agencé de manière à, lorsque le chariot 54 est en butée contre lui, coopérer avec la languette 66 et contraindre le deuxième ressort 68 de manière à ce que la languette laisse libre le décrochement 58. Lorsque le chariot 54 n'est pas en butée contre l'ergot 52, le deuxième ressort amène la languette 66 au-dessus du décrochement 58. On notera que, dans ce dernier cas, un espace permettant de loger la collerette 16 subsiste entre le décrochement 58 et la languette 66.

La deuxième cloison interne 62 présente un renvoi 70 sur lequel prend appui le premier ressort 56. Ce renvoi 70 est percé d'une ouverture susceptible d'être traversée par le prolongement 50 de la bague 44.

Les différentes fonctions des éléments qui ont été décrits ci-dessus vont maintenant apparaître plus clairement à la lecture de la description qui suit, faite en référence aux figures 1 à 9.

La figure 1 montre le dispositif d'auto-injection selon l'invention, dont le volet 26 est ouvert, afin de permettre l'insertion d'une seringue d'injection 10. La position du bras 42 en référence au volet 26, fait que les renflements 48 des crochets 46 de la bague 44 sont positionnés dans les premières creusures 36a. Le prolongement 50 de la bague 44 positionne la deuxième cloison interne 62 vers l'extrémité du volet 26 et le premier ressort 56 pousse le chariot 54 en butée contre l'ergot 52.

Lors de l'insertion de la seringue 10 dans le compartiment, la collerette 16 de la seringue vient prendre appui sur le décrochement 58 du chariot 54 et l'utilisateur, en contraignant le premier ressort 56, amène ensuite le bec 60 se bloquer sur l'échancrure 64. Lorsque le volet 26 commence à être rabattu (figure 2), l'ergot 52 ne coopère plus avec la languette 66, celle-ci est pressée par le deuxième ressort 68 et recouvre la collerette 16, empêchant la seringue 10 de sortir accidentellement du compartiment. Il est nécessaire de ramener totalement le chariot 54 en butée contre l'ergot 52 manuellement ou en ouvrant complètement le volet 26 pour libérer la seringue, éventuellement en l'enlevant de force, mais en tout cas de manière volontaire.

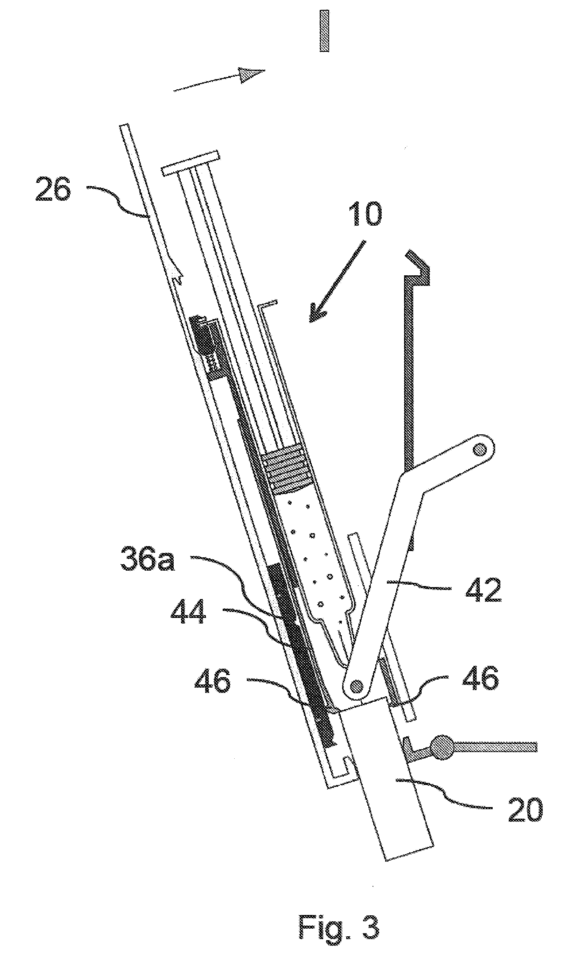

En poursuivant la fermeture du volet 26, le bras 42 pivote à ses deux extrémités et entraîne la bague 44 en translation. Les crochets 46 quittent les creusures 36a et se resserrent en direction de l'intérieur du compartiment de manière à venir coopérer avec le capuchon 20 de la seringue 10. En fonction de la configuration et de la disposition des crochets 46, cette coopération peut constituer en une poussée par le bord du capuchon 20. Les crochets peuvent également venir mordre dans le capuchon 20 de manière à le solidariser avec la bague 44. Avantageusement, on combine des crochets positionnés différemment, afin d'avoir une meilleure coopération entre eux et le capuchon 20 et de manière à pouvoir solidariser le capuchon sur la seringue en cas d'interruption de la fermeture. Ainsi, comme le montre la figure 3, la poursuite de la fermeture du volet permet de pousser le capuchon 20 et de le désolidariser de la seringue 10.

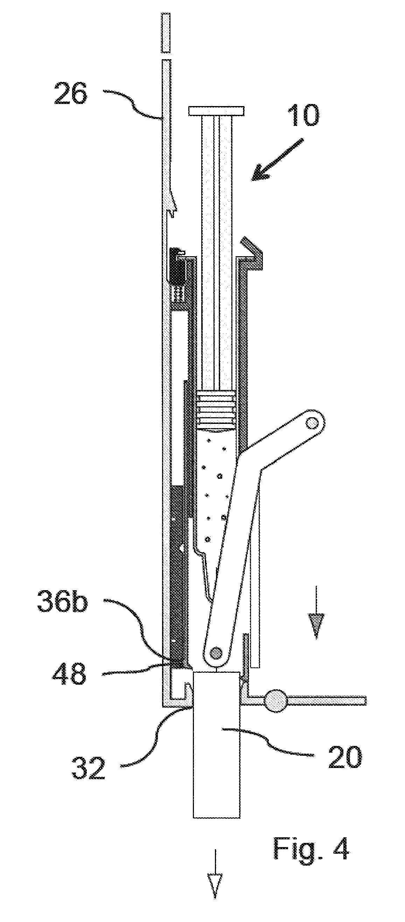

Lorsque le volet 26 est fermé (figure 4), les renflements 48 retombent dans les deuxièmes creusures 36b de manière à ne plus coopérer avec le capuchon 20. Celui-ci dépasse du dispositif par l'ouverture 32 qui peut avantageusement être dimensionnée de manière à légèrement retenir le capuchon 20, pour que celui-ci ne tombe pas. L'aiguille de la seringue reste en retrait de la surface extérieur du dispositif et l'ouverture suffisamment petite pour empêcher tout contact avec le patient. Le loquet du volet 26 est alors verrouillé, et il n'est pas possible de rouvrir le volet, tant que le capuchon n'est pas remis en place sur la seringue 10.

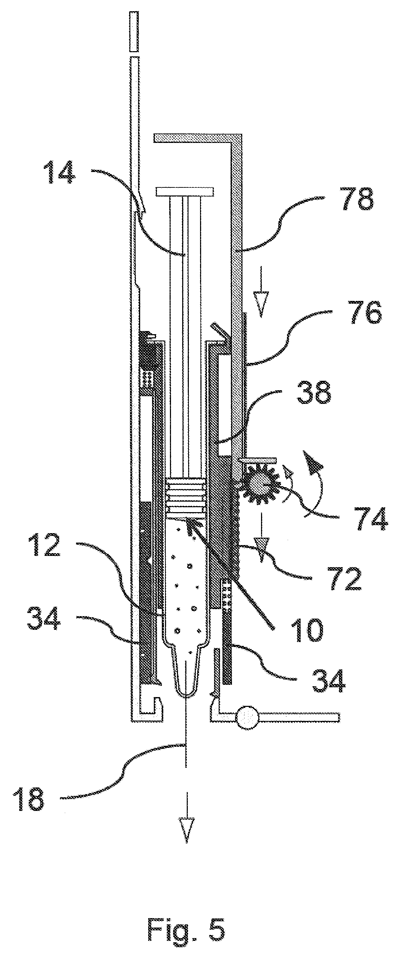

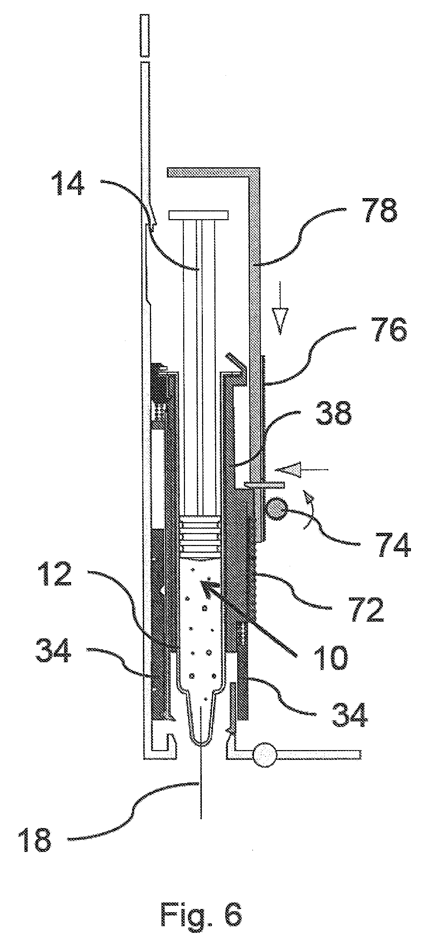

Le patient peut alors positionner le dispositif et déclencher l'injection (figures 5 et 6). La seringue 10 est déplacée dans le compartiment, en direction de l'ouverture 32, pour que l'aiguille 18 pénètre la peau du patient. Le piston 14 est actionné de manière à ce que tout ou partie du produit contenu dans le corps 12 soit injecté. Après l'injection, la seringue 10 est remontée à l'intérieur du dispositif et protégée de tout contact accidentel avec le patient ou une autre personne.

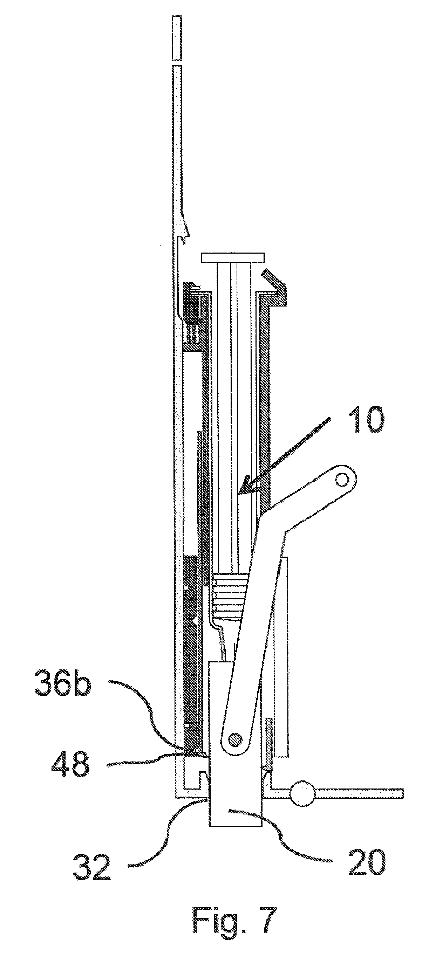

Les renflements 48 sont toujours dans les deuxièmes creusures 36b, de sorte que le patient peut librement insérer le capuchon 20 dans l'ouverture 32 et le solidariser sur la seringue 10, particulièrement après une injection. Le capteur détecte alors la présence du capuchon 20, ce qui déverrouille le loquet et autorise l'ouverture du volet (figure 7).

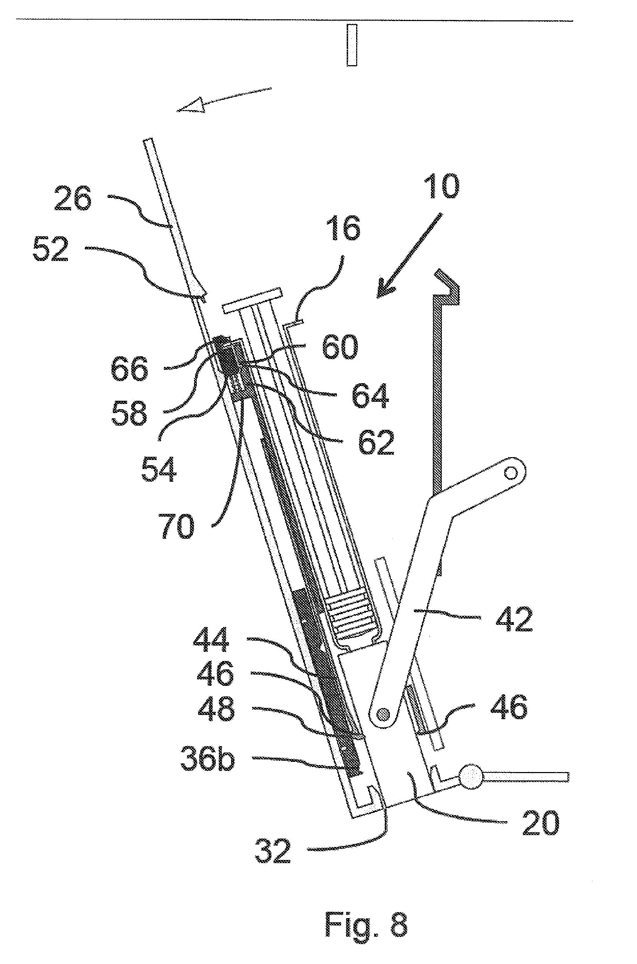

En ouvrant le volet (figure 8), typiquement par un bouton agissant sur le loquet, le mouvement du bras 42 ramène la bague 44 dans une direction opposée à l'ouverture 32. Ceci fait ressortir les renflements 48 de la deuxième creusure 36b et les crochets 46 viennent coopérer avec le capuchon 20, ce qui permet de garantir que celui-ci ne peut se désolidariser de la seringue 10, lors de l'extraction de celle-ci. On remarquera que, dans ce cas, tous les crochets 46 mordent dans le capuchon 20, car les renflements 48 sont situés dans les deuxièmes creusures 36b.

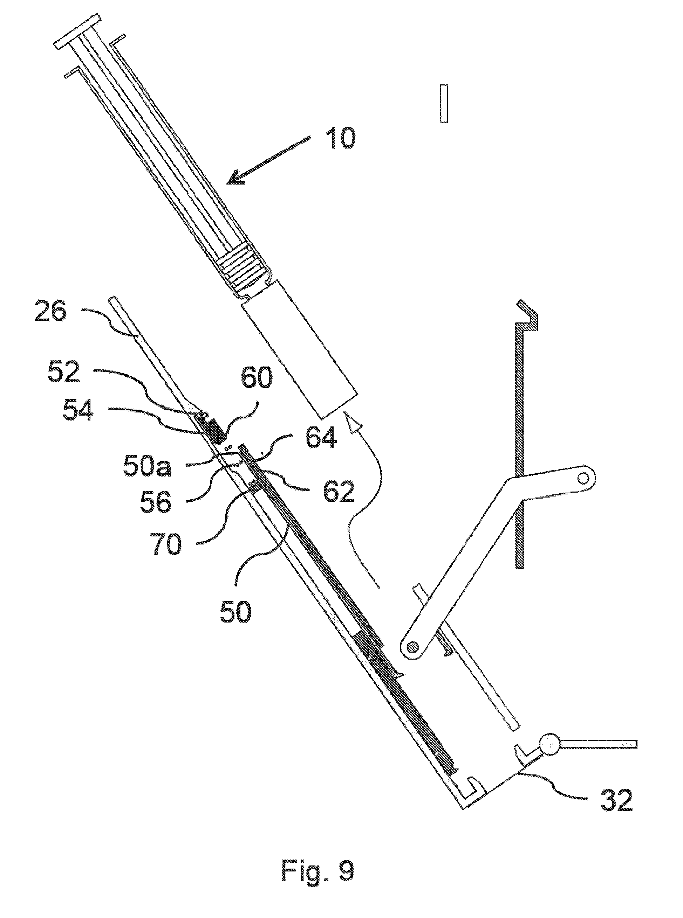

En poursuivant l'ouverture du volet 26, le doigt 50a du prolongement 50 traverse l'ouverture du renvoi 70 et écarte la deuxième cloison interne 62 de manière à libérer le bec 60 de l'échancrure 64. Le chariot 54 est alors librement soumis à l'action du premier ressort 56, qui le pousse en direction de l'ergot 52. Cela a pour effet d'amener la seringue 10 vers la sortie du compartiment, car la collerette 16 est encore prise entre le décrochement 58 et la languette 66. Enfin, lorsque le chariot 54 arrive en butée contre l'ergot 52, la languette 66 libère la collerette 16 et le patient peut sortir la seringue 10, protégée par son capuchon 20, en toute sécurité (figure 9).

Ainsi, le système de désolidarisation du capuchon 20 en référence à la seringue 10, coordonné avec la fermeture du volet 26, d'une part, et l'assujettissement du déverrouillage du loquet fermant le volet 26, d'autre part, permettent et garantissent que le patient insère et extraie la seringue avec son capuchon. Il est, de fait, impossible de se blesser avec l'aiguille, avant, pendant et après l'injection.

Le capteur détectant la présence du capuchon 20 peut être utilisé pour conditionner le déclenchement d'une injection au retrait de ce capuchon, de manière à ce qu'une injection ne puisse être déclenchée si le capuchon est toujours présent dans l'ouverture 32.

Pour la réalisation de l'injection, il est nécessaire, dans un premier temps, de déplacer toute la seringue en direction de l'ouverture, de manière à faire pénétrer l'aiguille dans la peau du patient. Dans un deuxième temps, il faut actionner le piston pour faire sortir le liquide contenu dans la seringue et l'injecter effectivement.

Pour la première étape, on peut voir, sur les figures 5 et 6, qu'une crémaillère 72 est montée solidairement à la cloison interne 38. Pour le passage de la crémaillère 72, une ouverture peut être ménagée dans l'anneau de guidage 34. Cette crémaillère coopère avec un premier niveau d'un pignon 74 entraîné par un moteur, non représenté.

Le pignon 74 comporte avantageusement un deuxième niveau, disposé de manière à coopérer avec une deuxième crémaillère 76 montée sur un pousse-piston 78. La première et la deuxième crémaillères sont agencées de manière à ce que, lors de la rotation du pignon 74, le premier puis le deuxième niveaux coopèrent successivement et respectivement avec les crémaillères 72 et 76. Ainsi sont successivement réalisées les première et deuxième étapes de l'injection. De manière avantageuse, un seul moteur est utilisé pour effectuer ces deux opérations. On peut éventuellement jouer sur les dentures des deux niveaux du pignon et des crémaillères ainsi que sur la vitesse de rotation du moteur pour adapter les vitesses d'entraînement.

Dans un deuxième mode de réalisation non représenté, les premières et les deuxièmes crémaillères peuvent être entraînées par deux moteurs différents. On relèvera également que le piston pourrait être entraîné directement par une vis sans fin coopérant avec le disque terminant généralement le piston, au lieu d'un entraînement par un pousse-seringue.

Le dispositif d'auto-injection selon l'invention est doté de moyens électroniques permettant de commander les différentes opérations effectuées au cours de l'injection, notamment la mise en fonction des moteurs pour la descente de la seringue et pour l'actionnement du piston. Les moyens électroniques sont également utilisés, comme mentionné ci-dessus, pour commander le verrouillage du loquet en fonction de la présence du capuchon sur la seringue.

Les moyens électroniques sont avantageusement programmables. La programmation peut se faire éventuellement par une interface directe, comportant un écran et des boutons de commande, ou par une interface indirecte, située à distance de l'appareil et communiquant avec le dispositif par Internet ou par liaison téléphonique.

Les moyens électroniques peuvent ainsi être programmés pour gérer des injections de doses, formées par des portions du contenu de la seringue. Dès lors que le dispositif est programmé de manière à connaître le produit présent dans la seringue et sa concentration, et la dose de médicament à administrer, le dispositif détermine la quantité de produit à injecter et, au moment de l'injection, l'administre. Le dispositif peut également intégrer un capteur électronique, par exemple optique, pour détecter la position du piston.

Les moyens électroniques peuvent, de manière avantageuse, être utilisés pour suivre et contrôler le bon suivi du traitement et, éventuellement, rappeler au patient le moment des injections. De préférence, les données relatives au patient et à son traitement son stockées à distance, sur un serveur avec lequel le dispositif communique.

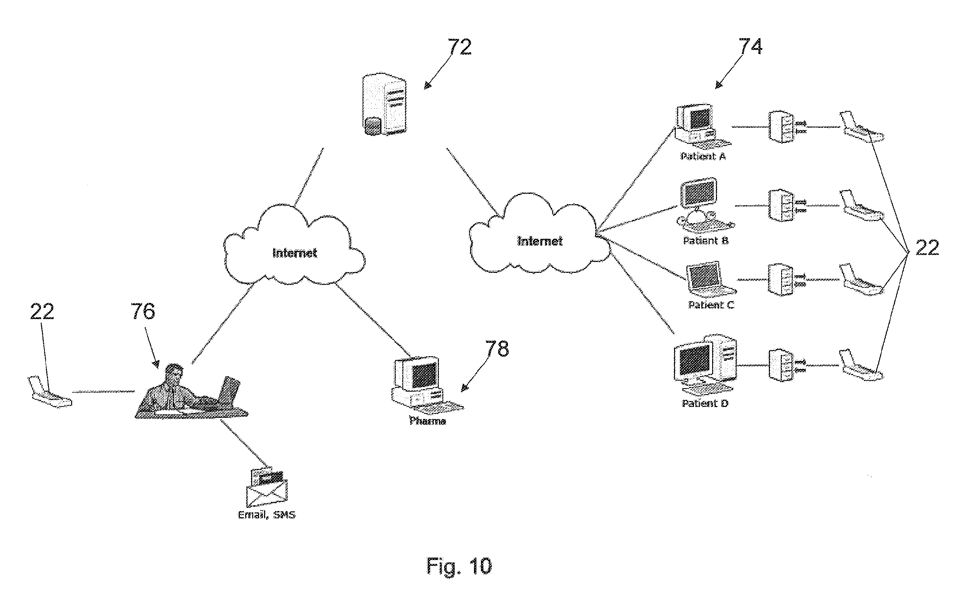

Ainsi, la figure 10 illustre un système de gestion et de contrôle de protocoles d'injection, comprenant :
- un serveur 72 stockant des données relatives au patient et à son traitement, et
- au moins un dispositif d'auto-injection 22 tel que décrit précédemment, comportant des moyens de connexion pour être relié à distance au serveur.

On a représenté sur la figure des dispositifs 22 pour des patients A, B, C et D.

Les moyens électroniques sont agencés pour :
- enregistrer l'historique des injections,
- suivre et contrôler le suivi du traitement et, éventuellement, rappeler au patient le moment des injections,
- transmettre au serveur ledit historique,
- recevoir et traiter des informations transmises par le serveur.

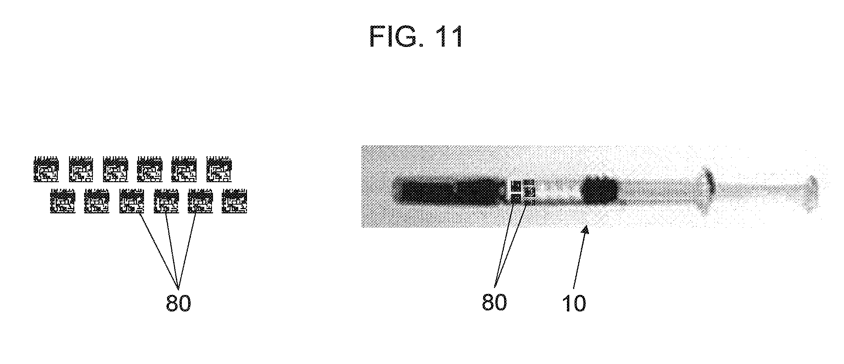

Dans des modes de réalisation préférés, les moyens électroniques peuvent, en outre, être programmés pour détecter et contrôler la nature et date limite d'utilisation de la seringue. Comme le montre la figure 11, ces informations peuvent être codées sous forme de codes barres 2D, prenant la forme d'une étiquette 80 de petite dimension, c'est-à-dire occupant une portion réduite de la seringue, notamment une portion réduite de sa circonférence. Un lecteur de ce type de code barre est prévu dans le dispositif d'auto-injection. Pour éviter tout problème d'orientation de la seringue par rapport au lecteur, une pluralité d'étiquettes 80 est disposée sur le pourtour de la seringue. Afin de réduire encore davantage le problème d'orientation, une pluralité d'étiquettes 80 est disposée en quinconce sur le pourtour, selon deux lignes annulaires. On pourrait prévoir davantage de lignes, les étiquettes étant décalées les une en référence aux autres, de sorte qu'aucune portion angulaire donnée du corps de la seringue ne comporte pas d'étiquette. Les lignes annulaires selon lesquelles sont disposées les étiquettes peuvent être contigües, de sorte que les étiquettes se touchent (ou presque) par leur coin, ou être séparées. Le dispositif comportant un ou plusieurs lecteurs pour lire les codes barres.

Ainsi, quelle que soit la position de la seringue dans le dispositif, le lecteur peut toujours lire au moins un code barre. Les codes barres peuvent être imprimés avec une encre invisible à la lumière du jour, mais sensible aux UV par exemple, une source lumineuse correspondante étant disposée dans le dispositif d'auto-injection pour rendre visible les codes barres.

En outre, les moyens électroniques peuvent aussi être utilisés pour réguler les paramètres d'injections de vitesse ou de temps. On peut encore relever que les moyens électroniques peuvent autoriser ou empêcher l'injection en fonction de la détection de l'absence de capuchon et de la présence de la peau.

Par exemple, on peut prévoir que le dispositif d'auto-injection se connecte au serveur par l'intermédiaire d'un téléphone portable, communicant sans fil avec le dispositif d'auto-injection. On peut également proposer que le dispositif doive être rechargé électriquement en étant branché à un ordinateur 74, par une connexion USB et que, à cette occasion, si l'ordinateur est connecté à Internet, le dispositif se relie automatiquement au serveur. Dans les deux cas, les moyens électroniques peuvent être programmés pour fournir au serveur un élément d'identification, qui permet de reconnaître le dispositif et le traitement associé au patient. Le serveur peut alors charger depuis le dispositif, l'historique des injections. Cet historique peut ensuite être consulté par le médecin 76, éventuellement lorsque celui-ci se connecte au serveur ou alors, en envoyant par email un fichier comportant l'historique. Le médecin peut, le cas échéant, modifier le programme d'injection du patient ou le dosage, ces modifications étant transmises au dispositif lors de sa prochaine connexion.

Les entreprises pharmaceutiques 78 peuvent également avoir la possibilité de se connecter au serveur 72, par exemple pour modifier des données relatives aux produits injectés ou prévenir de l'utilisation de certaines seringues identifiées par leur numéro de lot ou de série, notamment pour garantir qu'une seringue ne soit pas utilisée deux fois.

Les moyens électroniques peuvent être programmés pour fournir des signaux de rappel au patient, afin de l'avertir du moment auquel il doit effectuer la prochaine injection. Ces signaux peuvent être sonores, lumineux, ou consister en des messages affichés sur l'écran du dispositif. Les rappels peuvent aussi être envoyés au patient par le serveur, par email ou par SMS. En fonction des traitements et des maladies, le dispositif peut aussi envoyer un signal d'alarme lorsqu'une injection n'est pas faite ou après un certain délai pendant lequel le dispositif n'a pas été utilisé.

Naturellement, les différentes fonctions de communication du dispositif avec le serveur ou avec d'autres appareils peuvent être activées ou désactivées, soit par les fournisseurs de dispositif, soit par les médecins traitant les patients.

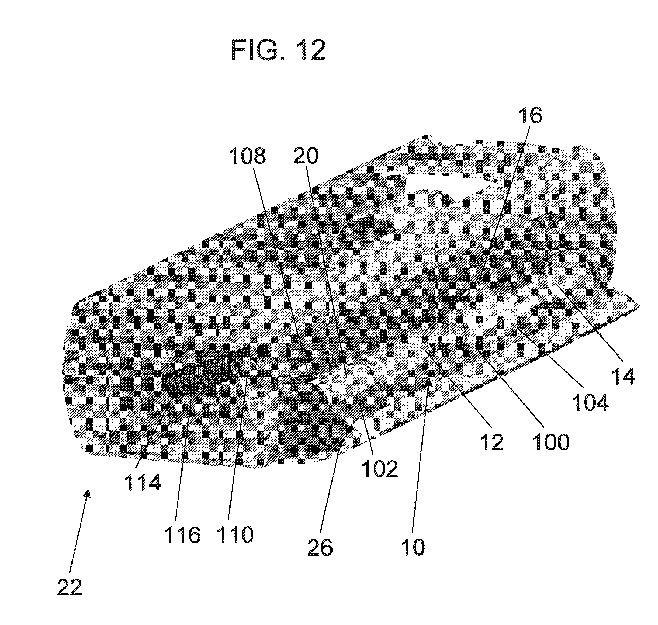

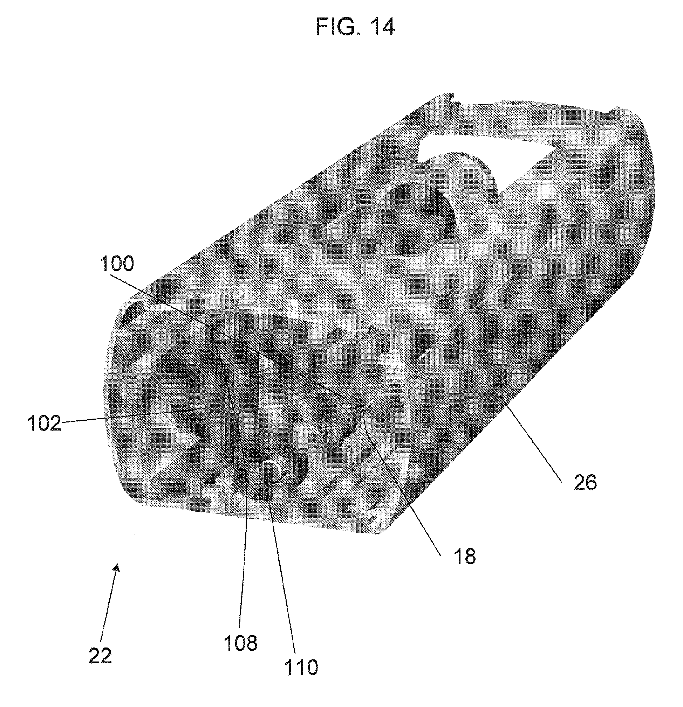

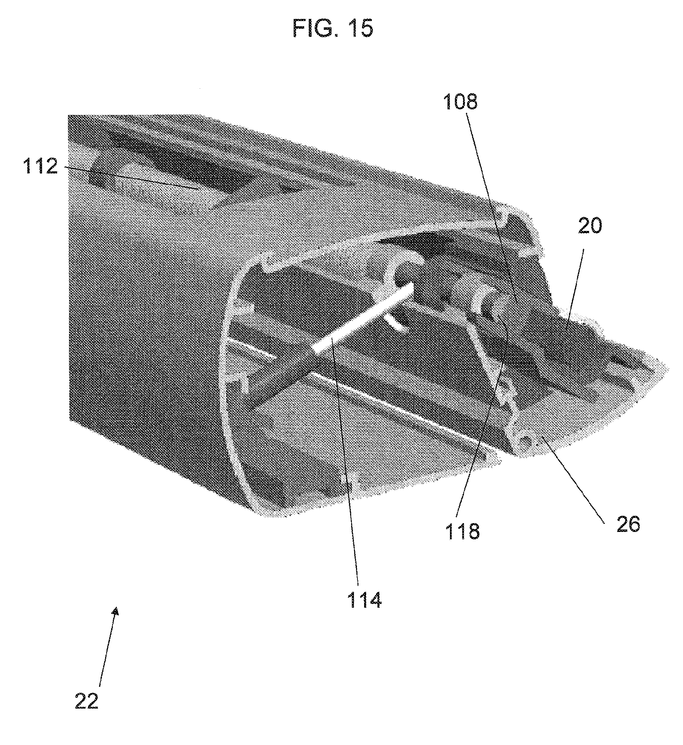

Les figures 12 à 15 se rapportent à un mode de réalisation supplémentaire d'un dispositif d'auto-injection 22 selon l'invention, dans lequel le retrait du capuchon est géré par les moyens électroniques, après la fermeture du volet 26.

Tout comme dans le premier mode de réalisation, le dispositif d'auto-injection 22 comprend un compartiment fermé par un volet 26 articulé. Le compartiment est susceptible d'évoluer entre une première position ouverte pour l'insertion et le retrait de la seringue 10, et une deuxième position fermée pour la réalisation de l'injection. Dans cette variante, le volet 26 s'ouvre latéralement en référence au dispositif, c'est-à-dire selon un axe parallèle à l'axe longitudinal du dispositif. L'intérieur du compartiment comprend un réceptacle en deux parties, en forme de gouttière, dont la courbure est sensiblement identique à celle des seringues conventionnelles, pour le type d'injection visé. Une première partie 100 est destinée à recevoir le corps 12 et le piston 14 de la seringue 10 lorsque celle-ci est introduite dans le compartiment, tandis qu'une deuxième partie 102 est destinée à recevoir le capuchon 20 protégeant l'aiguille 18.

La première partie 100 est structurée de manière à permettre une insertion précise et sans jeu de la seringue 10 dans le réceptacle. Un épaulement 104 est prévu pour fournir un appui à la collerette 16, afin de permettre le maintien du corps 12 lors de l'actionnement du piston 14. Typiquement, la première partie 100 comprend au moins une zone déformable élastiquement pour maintenir par clipsage le corps de la seringue 10. L'homme du métier peut prévoir d'autre système de maintien de la seringue, tel qu'un levier positionné par un ressort et maintenant la seringue en place. La première partie 100 peut, en outre, comporter un dispositif permettant d'actionner le piston 16 lors de l'injection.

La première partie 100 est montée mobile en translation en référence à la deuxième partie 102, à l'intérieur du dispositif. On peut, par exemple, utiliser un entraînement par pignon et crémaillère, du type de celui-décrit dans le premier mode de réalisation, ou une vis sans fin.

La deuxième partie 102 est montée mobile en rotation en référence à la première partie 100. La deuxième partie 102 est susceptible d'occuper une première position dans laquelle elle prolonge la première partie 100, de manière à ce qu'un utilisateur puisse introduire le corps 12 de la seringue 10 dans la première partie 100, tandis que le capuchon 20 se positionne au niveau de la deuxième partie 102. Particulièrement, la deuxième partie 102 est dans cette première position lorsque le compartiment est ouvert. Comme on le comprendra par la suite, la deuxième partie 102 est également susceptible d'occuper au moins une deuxième position dans laquelle elle est totalement située en dehors de la course en translation de la première partie 100.

La deuxième partie est munie d'un système de serrage du capuchon, comportant une pince 108 capable de coopérer avec le capuchon 20 en l'enserrant. La pince 108 est agencée de manière à laisser libre le capuchon 20 lorsque le volet 26 est ouvert, c'est-à-dire lorsque le compartiment et la deuxième partie 102 sont dans leur première position. Par ailleurs, la pince 108 est agencée pour être solidarisée avec le capuchon lorsque le volet est fermé. De préférence, le passage de la pince 108 de la position libre à la position enserrée, se fait de manière automatique.

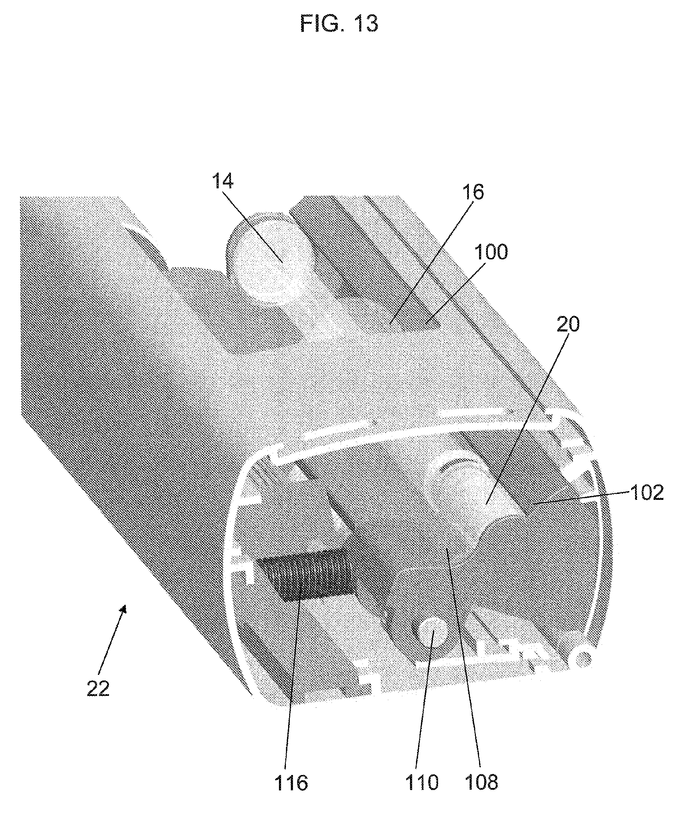

Les figures 12 à 15 illustrent la deuxième partie du réceptacle. On peut notamment voir un axe 110 autour duquel pivote la deuxième partie 102. Cet axe 110 est distinct de l'axe autour duquel pivote le volet 26. Une vis sans fin 112 est située dans le prolongement de l'axe 110, pour l'entraînement en translation de la première partie 100. La vis sans 112 est indépendante de l'axe 110. Un vérin 114 muni d'un ressort 116 (représenté seulement sur les figures 12 et 13) est interposé entre l'intérieur du dispositif d'auto-injection et l'axe 110. Le vérin 114 est monté articulé sur le dispositif d'auto-injection et sur l'axe 110, qu'il entraîne en rotation. Le vérin 114 et le ressort 116 sont agencés de manière à exercer sur l'axe 110 une force tendant à ouvrir le volet 26 du compartiment. L'ouverture et la fermeture du volet 26 entraînent la rotation de l'axe 110.

Dans l'exemple illustré, la pince 108 est susceptible d'évoluer en translation en référence à l'axe 110. Elle peut ainsi présenter une ouverture oblongue à l'intérieur de laquelle passe l'axe 110. Ce dernier présente une portion excentrique, tel qu'un méplat 118 au niveau de l'ouverture oblongue. La pince est maintenue appuyée sur le méplat par un ressort. Ainsi, en fonction de l'orientation relative du méplat en référence à l'ouverture oblongue, la pince se déplace en translation en référence à l'axe, de sorte que la rotation de ce dernier entraîne un déplacement de la pince 108 définissant les positions libre et enserrée. La pince peut comporter un tampon de caoutchouc pour améliorer le maintien du capuchon.

Dans ce deuxième mode de réalisation, lorsque le volet 26 est ouvert, l'utilisateur insère une seringue 10 dans le compartiment, comportant la première 100 et la deuxième 102 parties situées dans le prolongement l'une de l'autre, comme le montre la figure 12. La seringue 10 est clipsée sans jeu sur la première partie 100. En refermant le volet 26, la pince 108 enserre automatiquement le capuchon. Le ressort 116 est contraint et un système de verrouillage du volet permet de maintenir le compartiment en position fermée.

Puis, de manière automatique, la première partie 100 du réceptacle translate, tandis que la deuxième partie 102 reste fixe et le capuchon 20 est maintenu par la pince 108. De fait, le capuchon 20 se désolidarise du reste de la seringue. La première partie 100 translate jusqu'à ce que l'aiguille 18 soit totalement sortie du capuchon 20, définissant une position extrême de la seringue 10. La course en translation de la première partie 100 est donc supérieure à la longueur des parties logées dans le capuchon 18.

La deuxième partie 102 du réceptacle peut alors, en vue d'une injection, pivoter selon l'axe 110 jusqu'à sa deuxième position, de manière à dégager le capuchon 20 de la course de la seringue et de manière à ce que la deuxième partie 102 ne gêne pas le déplacement de la première partie. On notera que, dans sa rotation, la deuxième partie 102 entraîne la pince 108 et l'axe 110, la position relative de ces deux derniers éléments étant fixe, de manière à toujours maintenir le capuchon.

Lorsque la deuxième partie 102 du réceptacle a atteint sa deuxième position, l'injection peut être réalisée en déplaçant d'abord le corps 12 et le piston 14 de la seringue à l'aide de la vis sans fin, puis en déplaçant le piston 14, à l'instar de ce qui a été décrit en référence au premier mode de réalisation.

Après l'injection, le corps 12 et le piston 14 de la seringue remontent dans le dispositif, jusqu'à la position extrême définie précédemment. La deuxième partie 102 peut alors reprendre sa première position et se remettre dans le prolongement de la première partie 100, le capuchon 20 étant alors dans l'axe du corps de la seringue. La première partie 100 translate alors de manière à solidariser à nouveau le capuchon sur la seringue. Le volet 26 peut alors être déverrouillé et ouvert et la seringue déclipsée, avec le capuchon 20 qui protège l'aiguille 18.

L'homme du métier pourra aisément et sans qu'il soit besoin de les décrire, prévoir les moteurs et actuateurs pour les déplacements des différents éléments. Ces déplacements sont gérés par des moyens électroniques, comme décrit ci-dessus. On notera encore que ces opérations peuvent se faire automatiquement dès lors que le volet 26 est détecté comme fermé, mais elles peuvent aussi être déclenchées par pression sur un organe de commande.

Des capteurs et systèmes de sécurité électroniques ou électromécaniques peuvent être prévus de manière à garantir le bon déroulement des opérations, l'ensemble étant géré par lesdits moyens électroniques. Ainsi, la désolidarisation du capuchon est conditionnée par le passage du compartiment à sa position fermée et l'ouverture du compartiment est conditionnée par la solidarisation du capuchon. Plus particulièrement, les opérations du retrait du capuchon ne peuvent être réalisées que si un capteur détecte que le volet 26 est fermé et verrouillé. A l'inverse, le déverrouillage et l'ouverture du volet 26 ne sont autorisés que si un autre capteur confirme la présence du capuchon sur la seringue. En outre, le pivotement de la deuxième partie 102 n'est possible que si la première partie 100 est dans sa position extrême. La descente de la première partie 100 n'est possible que lorsque la deuxième partie 102 est dans sa deuxième position.

Ce mode de réalisation présente l'avantage que l'ensemble des opérations de retrait du capuchon se fait automatiquement, sans aucune intervention manuelle. Le capuchon demeure à l'intérieure du dispositif d'auto-injection, ce qui évite de le perdre lorsqu'il est enlevé.

On notera que le mode de réalisation décrit ci-dessus peut être adapté aisément, notamment en ce qui concerne les déplacements des première et deuxième parties du réceptacle. En effet, il est évident qu'il suffit que ces deux parties soient mobiles l'une en référence à l'autre, selon deux directions orthogonales, afin de permettre la (dé)solidarisation du capuchon et son dégagement/engagement. On pourrait ainsi envisager que les deux parties se déplacent selon deux mouvements de translation. Notamment dans ce dernier cas, le serrage du capuchon par la pince 108 peut être géré de manière électromécanique, c'est-à-dire qu'elle est commandée par la détection de la position verrouillée du volet 26 par un capteur, et que la libération du capuchon est déclenchée lors du déverrouillage du volet 26.

Ainsi est proposé un dispositif d'auto-injection perfectionné, destiné à être utilisé de manière répétée et permettant d'utiliser des seringues jetables, évitant que l'utilisateur doive manipuler la seringue avec son aiguille accessible. Le dispositif est également programmable, avantageusement à distance, pour un suivi aisé et efficace bilatéral, c'est-à-dire que tant le personnel soignant que le patient peut consulter, recevoir et transmettre des informations relatives au programme d'injections.

La description ci-dessus a été donnée à titre d'illustration non limitative de l'invention et l'homme du métier pourra prévoir d'autres modes de réalisation pour obtenir les fonctions décrites. On pourrait ainsi prévoir que le retrait du capuchon soit entièrement géré par les moyens électroniques, après la fermeture du volet.

## Revendications

1. Dispositif d'injection (22) doté d'un compartiment articulé en référence au dispositif pour recevoir une seringue (10) munie d'une aiguille (18) protégée par un capuchon (20) de protection solidaire de la seringue, le compartiment étant susceptible d'évoluer entre une première position ouverte pour l'insertion et le retrait de la seringue, et une deuxième position fermée pour la réalisation de l'injection,
**caractérisé en ce que** le dispositif comporte un système de désolidarisation destiné à coopérer avec le capuchon de la seringue pour le désolidariser de la seringue, la désolidarisation étant conditionnée par le passage du compartiment à sa deuxième position,
et **en ce qu'**il comporte des moyens agencés de manière à ce que le passage du compartiment de la deuxième à la première position est conditionné par la solidarisation du capuchon (20).

2. Dispositif selon la revendication 1, **caractérisé en ce que** la désolidarisation est effectuée automatiquement lors du passage de la première à la deuxième position.

3. Dispositif selon la revendication 2, **caractérisé en ce que** le système de désolidarisation comporte des crochets (46) agencés pour coopérer avec le capuchon (20), lesdits crochets (46) étant entraînés en translation en référence au capuchon (20), en fonction de la position du compartiment, entre une première et une deuxième positions, les crochets étant agencés de manière à ne pas coopérer avec le capuchon (20) lorsqu'ils sont dans les première et deuxième positions, et à coopérer avec le capuchon (20) lorsqu'ils sont entre les première et deuxième positions.

4. Dispositif selon l'une des revendications précédentes, **caractérisé en ce qu'**il est agencé de manière à laisser accessible le capuchon (20) après la désolidarisation, pour un retrait manuel, tandis que l'aiguille (18) demeure inaccessible à l'intérieur du dispositif.

5. Dispositif selon l'une des revendications précédentes, **caractérisé en ce qu'**il est agencé de manière à ce que, après une injection, l'aiguille (18) est disposée à l'intérieur du dispositif de manière inaccessible, le capuchon (20) pouvant être solidarisé à la seringue depuis l'extérieur.

6. Dispositif selon l'une des revendications précédentes, **caractérisé en ce qu'**il comporte un capteur agencé pour détecter la présence du capuchon (20) solidarisé sur la seringue (10), et **en ce que** le capteur régit le verrouillage du compartiment dans sa deuxième position, le compartiment étant maintenu verrouillé tant que le capteur ne détecte pas la présence du capuchon (20).

7. Dispositif selon l'une des revendications précédentes, dans lequel est défini une position d'ouverture maximale, **caractérisé en ce qu'**il comporte un système de verrouillage de la seringue (10) dans le compartiment, agencé de manière à ce que, lorsque la seringue (10) a été complètement insérée dans le compartiment, la seringue (10) soit verrouillée dans le compartiment tant que ce dernier n'est pas revenu à sa position d'ouverture maximale.

8. Dispositif selon la revendication 6, **caractérisé en ce que** le système de verrouillage de la seringue coopère avec un organe ressort (56) agencé de manière à pousser la seringue (10) en direction de la sortie du compartiment lorsque le compartiment atteint sa position d'ouverture maximale.

9. Dispositif selon la revendication 1, **caractérisé en ce que** le système de désolidarisation comporte un réceptacle en deux parties :
- une première partie (100) destinée à recevoir et à maintenir la seringue (10), et montée en translation en référence au dispositif,
- une deuxième partie (102) destinée à recevoir le capuchon (20), lesdites première et deuxième parties étant mobiles l'une en référence à l'autre, selon deux directions orthogonales,
**caractérisé en ce que** ladite deuxième partie est munie d'un système de serrage du capuchon, comportant une pince (108) agencée de manière à laisser libre le capuchon (20) lorsque le compartiment est dans sa première position, de manière à enserrer fermement le capuchon lorsque le compartiment est dans sa deuxième position.

10. Dispositif selon la revendication 9, **caractérisé en ce que** la pince (108) est montée sur un excentrique entraîné en rotation lors du passage du compartiment de l'une à l'autre de ses première et deuxième positions, la rotation de l'excentrique entraînant un déplacement de la pince (108) définissant les positions libre et enserrée de la pince.

11. Dispositif selon l'une des revendications 9 et 10, **caractérisé en ce que** lesdites première (100) et deuxième (102) parties sont mobiles en translation l'une en référence à l'autre selon une course supérieure à la longueur des parties logées dans le capuchon, de manière à ce que l'aiguille (18) soit totalement sortie du capuchon (20).

12. Dispositif selon la revendication 11, comportant des moyens électroniques et des actuateurs pour déplacer les première (100) et deuxième (102) parties du réceptacle, **caractérisé en ce que** les moyens électroniques sont programmés pour commander les actuateurs, de manière à, lorsqu'une seringue (10) est logée dans le compartiment et que ce dernier est dans sa première position, effectuer les étapes suivantes :
- translater les première (100) et deuxième (102) parties du réceptacle l'une en référence l'autre tandis que le capuchon (20) est maintenu par le système de serrage (108), le capuchon se désolidarisant du reste de la seringue, jusqu'à ce qu'à une position extrême dans laquelle l'aiguille (18) est totalement sortie du capuchon (20),
- déplacer les première (100) et deuxième (102) parties du réceptacle l'une en référence à l'autre de manière à dégager le capuchon (20) de la course de la seringue (10), en vue d'une injection,
- réaliser l'injection en déplaçant d'abord la seringue (10) puis en déplaçant son piston (14),
- après l'injection, ramener la seringue (10) à la position extrême,
- déplacer les première (100) et deuxième (102) parties du réceptacle l'une en référence à l'autre de manière à amener le capuchon (18) dans l'axe de la seringue (10),
- translater les première (100) et deuxième (102) parties du réceptacle l'une en référence à l'autre tandis que le capuchon (18) est maintenu par le système de serrage, le capuchon (18) se solidarisant à nouveau au reste de la seringue.

13. Système de gestion et de contrôle de protocoles d'injection, comprenant :
- un serveur (72) stockant des données relatives au patient et à son traitement, et
- au moins un dispositif d'injection (22) selon l'une des revendications précédentes pour réaliser les injections, comportant des moyens de connexion pour être relié à distance au serveur et des moyens électroniques,
**caractérisé en ce que** lesdits moyens électroniques sont agencés pour
- enregistrer l'historique des injections,
- suivre et contrôler le suivi du traitement et, éventuellement, rappeler au patient le moment des injections,
- transmettre au serveur ledit historique,
- recevoir et traiter des informations transmises par le serveur.

14. Système selon la revendication 13, **caractérisé en ce que** lesdits moyens électroniques sont en outre agencés pour détecter et contrôler la nature et date limite d'utilisation d'une seringue (10).

15. Système selon l'une des revendications 13 et 14, **caractérisé en ce que** lesdits moyens électroniques sont en outre agencés pour réguler les paramètres d'injections de vitesse ou de temps,

16. Système selon l'une des revendications 14 à 15, **caractérisé en ce que** les moyens de connexion sont agencés de manière à relier sans fil ledit dispositif à un téléphone portable, ledit téléphone portable reliant le dispositif audit serveur.

17. Système selon l'une des revendications 14 à 16, comprenant une seringue pour être utilisée par ledit dispositif d'injection, **caractérisé en ce que** ladite seringue porte une pluralité de codes barre 2D, disposés sur une ligne annulaire ou disposés en quinconce selon au moins deux lignes annulaires situées sur le pourtour de la seringue.

## Patentansprüche

1. Einspritzvorrichtung (22), die mit einem in Bezug auf die Vorrichtung angelenkten Fach zur Aufnahme einer Spritze (10) ausgestattet ist, die mit einer mit der Spritze fest verbundenen, von einer Schutzkappe (20) geschützten Nadel (18) ausgestattet ist, wobei das Fach imstande ist, sich zwischen einer ersten geöffneten Stellung für das Einsetzen und das Herausnehmen der Spritze und einer zweiten geschlossenen Stellung für die Durchführung der Injektion bewegbar ist,
**dadurch gekennzeichnet, dass** die Vorrichtung ein Lösesystem aufweist, das bestimmt ist, mit der Kappe der Spritze zusammenzuarbeiten, um sie von der Spritze zu lösen, wobei das Lösen von dem Übergang des Fachs in seine zweite Stellung bedingt ist,
und dass sie Mittel aufweist, die derart ausgebildet sind, dass der Übergang des Fachs von der zweiten in die erste Stellung von der festen Verbindung der Kappe (20) bedingt ist.

2. Vorrichtung nach Anspruch 1, **dadurch gekennzeichnet, dass** das Lösen beim Übergang von der ersten in die zweite Stellung automatisch erfolgt.

3. Vorrichtung nach Anspruch 2, **dadurch gekennzeichnet, dass** das Lösesystem Haken (46) aufweist, die ausgebildet sind, um mit der Kappe (20) zusammenzuarbeiten, wobei die Haken (46) in Bezug zur Kappe (20) in Abhängigkeit von der Stellung des Fachs zwischen einer ersten und einer zweiten Stellung in Verschiebung angetrieben werden, wobei die Haken derart ausgebildet sind, dass sie nicht mit der Kappe (20) zusammenarbeiten, wenn sie in der ersten und zweiten Stellung sind, und mit der Kappe (20) zusammenarbeiten, wenn sie zwischen der ersten und zweiten Stellung sind.

4. Vorrichtung nach einem der vorangehenden Ansprüche, **dadurch gekennzeichnet, dass** sie derart ausgebildet ist, dass die Kappe (20) nach dem Lösen für ein manuelles Entfernen zugänglich bleibt, wogegen die Nadel (18) unzugänglich im Innern der Vorrichtung verbleibt.

5. Vorrichtung nach einem der vorangehenden Ansprüche, **dadurch gekennzeichnet, dass** sie derart ausgebildet ist, dass die Nadel (18) nach einer Injektion unzugänglich im Innern der Vorrichtung angeordnet ist, wobei die Kappe (20) von außen mit der Spritze fest verbindbar ist.

6. Vorrichtung nach einem der vorangehenden Ansprüche, **dadurch gekennzeichnet, dass** sie einen Sensor aufweist, der ausgebildet ist, um die Anwesenheit der auf der Spritze (10) fest verbundenen Kappe (20) festzustellen, und dass der Sensor die Verriegelung des Fachs in seiner zweiten Stellung bestimmt, wobei das Fach solange verriegelt bleibt, wie der Sensor nicht die Anwesenheit der Kappe (20) feststellt.

7. Vorrichtung nach einem der vorangehenden Ansprüche, wobei eine maximale Öffnungsstellung definiert ist, **dadurch gekennzeichnet, dass** sie ein Verriegelungssystem der Spritze (10) in dem Fach aufweist, das derart ausgebildet ist, dass, wenn die Spritze (10) vollständig in das Fach eingesetzt wurde, die Spritze (10) in dem Fach verriegelt ist, solange dieses nicht in seine maximale Öffnungsstellung zurückgekehrt ist.

8. Vorrichtung nach Anspruch 6, **dadurch gekennzeichnet, dass** das Verriegelungssystem der Spritze mit einem Federorgan (56) zusammenarbeitet, das derart ausgebildet ist, dass die Spritze (10) in Richtung des Ausgangs des Fachs geschoben wird, wenn das Fach seine maximale Öffnungsstellung erreicht.

9. Vorrichtung nach Anspruch 1, **dadurch gekennzeichnet, dass** das Lösesystem einen Behälter aus zwei Teilen aufweist:
- einen ersten Teil (100), der bestimmt ist, die Spritze (10) aufzunehmen und zu halten, und der in Bezug auf die Vorrichtung in Verschiebung montiert ist,
- einen zweiten Teil (102), der bestimmt ist, die Kappe (20) aufzunehmen,
wobei der erste und zweite Teil in Bezug zueinander in zwei orthogonalen Richtungen bewegbar sind,
**dadurch gekennzeichnet, dass** der zweite Teil mit einem Spannsystem der Kappe ausgestattet ist, das eine Zange (108) aufweist, die derart ausgebildet ist, dass die Kappe (20) frei bleibt, wenn das Fach in seiner ersten Stellung ist, und derart, um die Kappe festzuspannen, wenn das Fach in seiner zweiten Stellung ist.

10. Vorrichtung nach Anspruch 9, **dadurch gekennzeichnet, dass** die Zange (108) auf einem Exzenter montiert ist, der beim Übergang des Fachs von der einen in die andere seiner ersten und zweite Stellung rotierend angetrieben wird, wobei die Rotation des Exzenters eine Verlagerung der Zange (108) bewirkt, die die freie und gespannte Position der Zange definiert.

11. Vorrichtung nach einem der Ansprüche 9 und 10, **dadurch gekennzeichnet, dass** der erste (100) und zweite (102) Teil in Bezug zueinander gemäß einem Weg in Verschiebung bewegbar sind, der länger ist als die Länge der Teile, die in der Kappe untergebracht sind, so dass die Nadel (18) vollständig aus der Kappe (20) heraustritt.

12. Vorrichtung nach Anspruch 11, die elektronische Mittel und Aktuatoren aufweist, um den ersten (100) und zweiten (102) Teil des Behälters zu verlagern, **dadurch gekennzeichnet, dass** die elektronischen Mittel programmiert sind, um die Aktuatoren zu steuern, so dass, wenn eine Spritze (10) in dem Fach aufgenommen ist und dieses in seiner ersten Stellung ist, die folgenden Schritte durchgeführt werden:
- Verschieben des ersten (100) und zweiten (102) Teils des Behälters in Bezug zueinander, wogegen die Kappe (20) von dem Spannsystem (108) gehalten wird, wobei sich die Kappe vom Rest der Spritze bis zu einer extremen Stellung löst, in welcher die Nadel (18) vollständig aus der Kappe (20) herausgetreten ist,
- Verlagern des ersten (100) und zweiten (102) Teils des Behälters in Bezug zueinander, um die Kappe (20) vom Weg der Spritze (10) zwecks einer Injektion zu entfernen,
- Durchführen der Injektion durch Verlagern zunächst der Spritze (10), danach durch Verlagern ihres Kolbens (14),
- nach der Injektion, Zurückführen der Spritze (10) in die extreme Stellung,
- Verlagern des ersten (100) und zweiten (102) Teils des Behälters in Bezug zueinander, um die Kappe (18) in die Achse der Spritze (10) zu führen,
- Verschieben des ersten (100) und zweiten (102) Teils des Behälters in Bezug zueinander, wogegen die Kappe (18) von dem Spannsystem gehalten wird, wobei sich die Kappe (18) mit dem Rest der Spritze erneut fest verbindet.

13. Management- und Kontrollsystem von Einspritzprotokollen, umfassend:
- einen Server (72), der Daten in Bezug auf den Patienten und auf seine Behandlung speichert, und
- mindestens eine Einspritzvorrichtung (22) nach einem der vorangehenden Ansprüche zur Durchführung von den Injektionen, die Verbindungsmittel aufweist, um aus der Ferne mit dem Server verbunden zu sein, und elektronische Mittel,
**dadurch gekennzeichnet, dass** die elektronischen Mittel ausgebildet sind, um:
- den Verlauf der Injektionen zu speichern,
- die Durchführung der Behandlung zu begleiten und zu kontrollieren und eventuell den Patienten an den Zeitpunkt der Injektionen zu erinnern,
- den Verlauf an den Server zu übertragen,
- die von dem Server übertragenen Informationen zu erhalten und zu verarbeiten.

14. System nach Anspruch 13, **dadurch gekennzeichnet, dass** die elektronischen Mittel ferner ausgebildet sind, um die Art und das Haltbarkeitsdatum einer Spritze (10) festzustellen und zu kontrollieren.

15. System nach einem der Ansprüche 13 und 14, **dadurch gekennzeichnet, dass** die elektronischen Mittel ferner ausgebildet sind, um die Einspritzparameter Geschwindigkeit oder Zeit einzustellen.

16. System nach einem der Ansprüche 14 und 15, **dadurch gekennzeichnet, dass** die Verbindungsmittel derart ausgebildet sind, um die Vorrichtung mit einem tragbaren Telefon drahtlos zu verbinden, wobei das tragbare Telefon die Vorrichtung mit dem Server verbindet.

17. System nach einem der Ansprüche 14 bis 16, das eine Spritze umfasst, um von der Einspritzvorrichtung verwendet zu werden, **dadurch gekennzeichnet, dass** die Spritze eine Vielzahl von 2D-Barcodes trägt, die auf einer ringförmigen Linie angeordnet sind oder beliebig gemäß mindestens zwei ringförmigen Linien angeordnet sind, die sich auf dem Umfang der Spritze befinden.

## Claims

1. An injection device (22) provided with a compartment articulated in reference to the device to receive a syringe (10) provided with a needle (18) protected by a protective cap (20) secured to the syringe, the compartment being able to move between a first open position for the insertion and removal of the syringe, and a second closed position for performing the injection,
**characterized in that** the device includes a separating system designed to cooperate with the cap of the syringe to separate it from the syringe, the separation being subject to the passage of the compartment to its second position,
and **in that** it includes means arranged so that the passage of the compartment from the second to the first position is subject to the securing of the cap (20).

2. The device according to claim 1, **characterized in that** separation is done automatically during the passage from the first to the second position.

3. The device according to claim 2, **characterized in that** the separating system includes hooks (46) arranged to cooperate with the cap (20), said hooks (46) being translated in reference to the cap (20), based on the position of the compartment, between first and second positions, the hooks being arranged so as not to cooperate with the cap (20) when they are in the first and second positions, and to cooperate with the cap (20) when they are between the first and second positions.

4. The device according to one of the preceding claims, **characterized in that** it is arranged so as to leave the cap (20) accessible after the separation, for manual removal, while the needle (18) remains inaccessible inside the device.

5. The device according to one of the preceding claims, **characterized in that** it is arranged such that, after injection, the needle (18) is positioned inside the device inaccessibly, the cap (20) being able to be secured to the syringe from the outside.

6. The device according to one of the preceding claims, **characterized in that** it includes a sensor arranged to detect the presence of the cap (20) secured to the syringe (10), and **in that** the sensor governs the locking of the compartment in its second position, the compartment being kept locked as long as the sensor does not detect the presence of the cap (20).

7. The device according to one of the preceding claims, wherein a maximum open position is defined, **characterized in that** it includes a system for locking the syringe (10) in the compartment, arranged such that, when the syringe (10) has been completely inserted in the compartment, the syringe (10) is locked in the compartment as long as the latter has not returned to its maximum open position.

8. The device according to claim 6, **characterized in that** the locking system of the syringe cooperates with a spring member (56) arranged so as to push the syringe (10) toward the outlet of the compartment when the compartment reaches its maximum open position.

9. The device according to claim 1, **characterized in that** the separating system includes a two-part receptacle:
- a first part (100) designed to receive and maintain the syringe (10), and mounted in translation in reference to the device,
- a second part (102) designed to receive the cap (20),
said first and second parts being movable in reference to one another, in two orthogonal directions,
**characterized in that** said second part is provided with a tightening system of the cap, including a clamp (108) arranged so as to leave the cap (20) free when the compartment is in its first position, so as to firmly grip the cap when the compartment is in its second position.

10. The device according to claim 9, **characterized in that** the clamp (108) is mounted on an eccentric disc rotated during the passage of the compartment from one to the other of its first and second positions, the rotation of the eccentric disc driving a movement of the clamp (108) defining the free and gripped positions of the clamp.

11. The device according to one of claims 9 and 10, **characterized in that** said first (100) and second (102) parts are translatable relative to one another along a travel exceeding the length of the parts housed in the cap, such that the needle (18) is completely outside the cap (20).

12. The device according to claim 11, including electronic means and actuators for moving the first (100) and second (102) parts of the receptacle, **characterized in that** the electronic means are programmed to command the actuator so as, when the syringe (10) is housed in the compartment and the latter is in its first position, to perform the following steps:
- translating the first (100) and second (102) parts of the receptacle in reference to one another while the cap (20) is maintained by the gripping system (108), the cap separating from the rest of the syringe, to an end position in which the needle (18) is completely outside the cap (20),
- moving the first (100) and second (102) parts of the receptacle in reference to one another so as to free the cap (20) from the travel of the syringe (10), for an injection,
- performing the injection by first moving the syringe (10), then moving its plunger (14),
- after the injection, returning the syringe (10) to the end position,
- moving the first (100) and second (102) parts of the receptacle in reference to one another so as to bring the cap (18) into the axis of the syringe (10),
- translating the first (100) and second (102) parts of the receptacle in reference to one another while the cap (18) is maintained by the gripping system, the cap (18) once again being secured to the rest of the syringe.

13. A system for managing and controlling injection protocols, comprising:
- a server (72) storing data relative to the patient and his treatment, and
- at least one injection device (22) according to one of the preceding claims for performing the injections, including connecting means to be connected remotely to the server and electronic means,
**characterized in** these electronic means are arranged to
- record the injection history,
- monitor and control the follow-up of the treatment and, optionally, remind the patient of injection times,
- send said history to the server,
- receive and process information sent by the server.

14. The system according to claim 13, **characterized in that** electronic means are further arranged to detect and control the nature and expiration date of the syringe (10).

15. The system according to one of claims 13 and 14, **characterized in that** said electronic means are further arranged to regulate the speed or time injection parameters.

16. The system according to one of claims 14 to 15, **characterized in that** the connecting means are arranged so as to wirelessly connect the device to a mobile telephone, said mobile telephone connecting the device to said server.

17. The system according to one of claims 14 to 16, comprising a syringe to be used by said injection device, **characterized in that** said syringe bears a plurality of 2D barcodes, positioned on an annular line or positioned in staggered rows in at least two annular lines situated on the perimeter of the syringe.
